# EUROPÄISCHE PATENTSCHRIFT

(11) **EP 3 223 890 B1**
(45) Veröffentlichungstag und Bekanntmachung des Hinweises auf die Patenterteilung: **05.12.2018**
(21) Anmeldenummer: 15774852.6
(22) Anmeldetag: 29.07.2015
(51) Int. Cl.: A61M 15/00

(54) **EINZELDOSIS-PULVERINHALATOR UND VERFAHREN ZU DESSEN HERSTELLUNG**
SINGLE-DOSE POWDER INHALATOR AND METHOD FOR THE PRODUCTION THEREOF
INHALATEUR DE POUDRE À DOSE UNITAIRE ET SON PROCÉDÉ DE FABRICATION

(30) Priorität: 26.11.2014 DE 102014017409
(43) Veröffentlichungstag der Anmeldung: 04.10.2017
(73) Patentinhaber: Perlen Converting AG, 6035 Perlen (CH); Beller, Klaus-Dieter, 79341 Kenzingen (DE)
(72) Erfinder: Beller, Klaus-Dieter, 79341 Kenzingen (DE)
(74) Vertreter: Mehl-Mikus, Claudia
(86) Internationale Anmeldenummer: PCT/EP2015/001558
(87) Internationale Veröffentlichungsnummer: WO 2016/082900

(56) Entgegenhaltungen:
- WO-A2-2007/042822
- WO-A2-2012/004485
- WO-A2-2012/047182
- WO-A2-2013/036881

## Beschreibung

Die Erfindung betrifft einen Einzeldosis-Pulverinhalator nach dem Oberbegriff des Anspruchs 1 und ein Verfahren zu dessen Herstellung.

Die am Markt üblichen Pulverinhalatoren ("Inhalator") sind häufig zur Mehrfachanwendung konzipiert und daher aufwändig und teuer in der Herstellung. Die Anwendung als Einweg-Artikel ist oft nicht geeignet. Die Mehrfachanwendung schafft aber zum Teil große hygienische Probleme, da der Patient den Inhalator nach dem Gebrauch nicht korrekt, selten oder gar nicht reinigt. Problematisch ist auch, dass Abdeck-Kappen verloren gehen. Es ist aus hygienischer Sicht auch ungünstig, wenn der Patient den Inhalator falsch benutzt, etwa in den Inhalator ausatmet: Bei bekannten Inhalatoren atmen Patienten nicht immer in vorgegebener Expirationsrichtung, sondern durch den Inhalator aus, sodass der Inhalator infolge der Atemluftfeuchtigkeit bei der Mehrfachanwendung zu Verstopfung neigt, da das pulverförmige Arzneimittel verklumpt und im Inhalator im Luftkanal kleben bleibt. Diese Ablagerungen und Verklebungen, die auch mit pathologischen Keimen belastet sein können, können zu Dosierungenauigkeiten und weiteren Nachteilen führen, etwa auch bei der nasalen Applikation: Dort sind Nasenkeime zu berücksichtigen, z. B. wenn sich eine Nasennebenhöhlenentzündung als therapieresistent erweist. Möglicherweise steckt sich der Betroffene über den Inhalator (nasal, oropharyngeal) immer wieder selbst an.

Die WO2007/042822 beschreibt einen Einzeldosis-Pulverinhalator, der ein erstes Gehäuseteil und ein zweites Gehäuseteil aus Metall aufweist, die zusammengefügt ein Inhalatorgehäuse mit Auslass bilden. Das erste Gehäuseteil umfasst eine Medikamentenkammer, die eine Einzeldosis eines pulverförmigen Medikaments enthält und die durch eine an dem ersten Gehäuseteil befestigte Folie verschlossen ist. Diese Folie erstreckt sich aus dem Inhalatorgehäuse, sodass sie zur Verwendung durch einen Anwender gegriffen und abgezogen werden kann, wodurch das pulverförmige Medikament aus der Medikamentenkammer freigesetzt wird. Das zweite Gehäuseteil weist einen Sammelschacht und/oder eine Dispersionskammer auf, durch die ein in dem Inhalatorgehäuse ausgebildeter Luftkanal zu dem Auslass führt. Nach Öffnung der Medikamentenkammer wird bei Inhalation am Auslass durch den Spalt zwischen den Gehäuseteilen, der nach dem Abziehen der Folie verbleibt, ein Luftstrom erzeugt, der das aus der Medikamentenkammer freigesetzte pulverförmige Medikament aus dem Sammelschacht bzw. der Dispersionskammer mitreißt.

WO2013/036881 beschreibt auch einen Einzeldosis-Pulverinhalator, wobei das Inhalatorgehäuse einen Einlasskanal und einen Auslasskanal aufweist. Von diesem Stand der Technik ist es Aufgabe der vorliegenden Erfindung, einen Einzeldosis-Pulverinhalator, der einfach und kostengünstig herstellbar, einfach In der Anwendung ist und hinsichtlich Desagglomeration des enthaltenen Pulvers und Vollständigkeit der Pulverentleerung verbessert ist.

Diese Aufgabe wird durch einen Einzeldosis-Pulverinhalator mit den Merkmalen des Anspruchs 1 gelöst.

Die Aufgabe der Herstellung eines solchen Inhalators wird durch das Verfahren mit den Merkmalen des unabhängigen Anspruchs 13 gelöst.

Weiterbildungen der Vorrichtung sind In den Unteransprüchen ausgeführt.

Ein erfindungsgemäßer Einzeldosis-Pulverinhalator besteht aus einem Inhalatorgehäuse, das ein Gehäuseteil aufweist, in dem zumindest eine Medikamentenkammer ausgeformt ist, die eine Gesamtdosis oder eine Teildosis eines pulverfömigen Medikaments enthält. Es handelt sich bei dem erfindungsgemäßen Einzeldosis-Pulverinhalator also quasi um einen blisterfrei, also ohne Einsatz eines separaten, mit Medikament befüllten Blisters, einsetzbaren Inhalator. Das Inhalatorgehäuse weist eine Auslassöffnung und einen Auslasskanal auf, der sich von der Medikamentenkammer zu der Auslassöffnung erstreckt.

Erfindungsgemäß ist das Inhalatorgehäuse zweiteilig, und weist ein Gehäuseteil und ein Deckelungselement auf, das mit dem Gehäuseteil zusammengefügt das Inhalatorgehäuse bildet. Dabei ist nicht nur auch der Auslasskanal in dem Gehäuseteil ausgeformt, sondern auch eine Lufteinlassöffnung auf einer von der Auslassöffnung abgewandten Seite der Medikamentenkammer und ein Einlasskanal, der sich von der Lufteinlassöffnung zu der Medikamentenkammer erstreckt, wobei der Auslasskanal, die Medikamentenkammer und der Einlasskanal eine Achse definieren.

So können sämtliche für den Pulverinhalator relevanten Abschnitte in einem einzigen Gehäuseteil ausgeformt sein, das dann lediglich nach Befüllung der Medikamentenkammer mit einer Pulverdosis mit einem

Deckelungselement gedeckelt wird. Um in der Anwendung des Einzeldosis-Pulverinhalators die Desagglomeration, die gleichmäßige Dispersion sowie die vollständige Entleerung des Pulvers zu unterstützen und zu verbessern, sind in dem Gehäuseteil in dem Lufteinlasskanal, in der Medikamentenkammer und in dem Auslasskanal Luftleit-, Verwirbelungs- und/oder Umlenkstrukturen ausgeformt, um die Luftströmung in, durch und aus der Medikamentenkammer zu beeinflussen. Zur Steuerung der Strömungsgeschwindigkeit ist zudem vorgesehen, dass sich der Einlasskanal von der Lufteinlassöffnung zu der Medikamentenkammer in zwei Richtungen senkrecht zu der durch den Auslasskanal, die Medikamentenkammer und den Einlasskanal definierten Achse verjüngt und der Auslasskanal von der Medikamentenkammer in Richtung der Auslassöffnung in zwei Richtungen senkrecht zu der durch den Auslasskanal, die Medikamentenkammer und den Einlasskanal definierten Achse aufweitet.

Hierdurch liegen vorteilhaft alle für einen optimal funktionierenden Pulverinhalator erforderlichen Strukturen in einem Gehäuseteil vor. D. h., der erfindungsgemäße Pulverinhalator besteht vorteilhaft einfach aus den genannten, lediglich zwei Gehäuseteilen, die zudem in einfacher Weise gefertigt und zusammengefügt werden können. Der Pulverinhalator lässt sich daher äußerst kostengünstig fertigen und ist somit zur Verabreichung einer Pulverdosis (oder auch einer Kombination aus mehreren Pulverdosen) in einer Einmalanwendung mit den damit verbundenen hygienischen Vorteilen für den Anwender geeignet. Ein erfindungsgemäßer Pulverinhalator stellt somit nicht nur die für eine optimale inhalative Verabreichung eines pulverförmigen Medikaments erforderlichen Strukturen bereit, sondern stellt auch eine Blisterverpackung für das pulverförmige Medikament dar.

Bevorzugt kann das Gehäuseteil kostengünstig aus Kunststoff bestehen und im Spritzgussprozess oder vorzugsweise durch Formung, besonders bevorzugt durch Tiefziehen, einer pharmagerechten, bevorzugt sortenreinen Kunststofffolie gefertigt werden. Zur Deckelung des Gehäuseteils, um den Lufteinlasskanal, die Medikamentenkammer und den Auslasskanal zu begrenzen und das Gehäuseteil zu dem Inhalatorgehäuse zu ergänzen, ist ein Deckelungselement vorgesehen, das einfach und kostengünstig flächig und im Wesentlichen planar und ungeformt ausgebildet sein kann. "Im Wesentlichen planar und ungeformt" soll hier bedeuten, dass das Deckelungselement in der einfachsten und bevorzugten Variante komplett eben sein kann; es kann jedoch auch vorgesehen sein, dass das Deckelungselement beispielsweise eine Längswölbung entlang dem Lufteinlasskanal, der Medikamentenkammer und dem Auslasskanal einerseits zur Stabilisierung und andererseits zur Strömungsoptimierung aufweist. Alternativ kann das Deckelungselement aber auch zumindest im Bereich des Einlasskanals und des Auslasskanals korrespondierend zu dem Gehäuseteil ausgeformt sein. Wie dieses kann das Deckelungselement daher ebenfalls aus Kunststoff bestehen und im Spritzgussprozess oder vorzugsweise durch Formung, besonders bevorzugt durch Tiefziehen, einer pharmagerechten, bevorzugt sortenreinen Kunststofffolie gefertigt werden. Der Aufbau eines erfindungsgemäßen Pulverinhalators entspricht somit dem einer Blisterverpackung. Dabei kann zumindest eines der beiden Inhalatorgehäusekomponenten Gehäuseteil und Deckelungselement transparent ausgeführt sein, sodass das in der Medikamentenkammer vorliegende Pulver und dessen Verabreichung durch Ansicht kontrolliert werden kann. Vorzugsweise kann daher zumindest das Gehäuseteil aus einer transparenten Kunststofffolie geformt sein, die mit einer Aluminium- oder Kunststofffolie gedeckelt ist, sodass lediglich Lufteinlass- und Auslassöffnung offen bleiben. Wird zur Deckelung eine Kunststofffolie verwendet, so kann diese vorzugsweise aus dem gleichen Kunststoff sein wie das erste Gehäuseteil, sodass der Pulverinhalator nach Gebrauch gut recycelt werden kann. Selbstverständlich kann als Deckelungselement auch eine Verbundfolie verwendet werden; dies ist aber aufgrund der schlechteren Recyclebarkeit nicht bevorzugt. Die Materialwahl für das Deckelungselement hängt auch davon ab, ob diese ein geformtes oder ein im wesentliches planares Deckelungselement sein soll. Ist das Deckelungselement geformt, so ist das bevorzugte Material eine Kunststofftiefziehfolie entsprechend dem Gehäuseteil, sodass das geformte Deckelungselement formstabil ist.

Das Deckelungselement lässt sich zudem vorteilhaft bedrucken, um dem Anwender Informationen über den Inhalt und Gebrauch des Inhalators bereitzustellen. Diese Information kann durch einen Text bereitgestellt werden oder vorzugsweise bildlich und gegebenenfalls farblich unterstützt sein, um international verständlich zu sein. Gegebenenfalls können Informationen auch in Blindenschrift (Brailleschrift) in die Folie eingebracht sein.

Es kann aber auch vorgesehen sein, dass das Gehäuseteil aus einem opaken oder eingefärbten Kunststoff hergestellt ist Auf diese Welse können unterschiedlich eingefärbte Kunststoffe auch eine Information über Art und/oder Menge des enthaltenen Pulvermedikaments bereitstellen.

Die Auslassöffnung kann als Mundrohr ausgeformt oder mit einem Nasenrüssel verbunden sein. Neben dem sich von der Lufteinlassöffnung zu der Medikamentenkammer verjüngenden Einlasskanal und dem sich von der Medikamentenkammer in Richtung der Auslassöffnung aufweitenden Auslasskanal (der sich gegebenenfalls bei Vorhandensein eines Nasenrüssels wieder verjüngt), kann zur Steuerung der Strömungsgeschwindigkeit ferner ein Querschnitt des Lufteinlasskanals an der Medikamentenkammer vorzugsweise kleiner gehalten sein als ein Querschnitt das Auslasskanals an der Medikamentenkammer.

Zur Unterstützung der Desagglomeration des Pulvers in der Medikamentenkammer in der Anwendung des Einzeldosis-Pulverinhalators kann dort zumindest ein loser Desagglomerator angeordnet sein, der von der turbulenten Luftströmung, die durch Gestaltung des Lufteinlasskanals in der Medikamentenkammer erzeugt wird, mit dem Pulver aufgewirbelt wird und Pulveragglomerate zerschlägt. Vorzugsweise wird ein Desagglomerator so groß bemessen, dass er in der Medikamentenkammer verbleibt und weder in den Lufteinlasskanal noch in den Auslasskanal dringen kann. Gegebenenfalls kann der Desagglomerator mit Durchbrüchen, d. h. luftdurchlässig ausgeführt sein, sodass ein Luftstrom nicht blockiert wird, auch wenn der Desagglomerator vor der Auslassöffnung der Medikamentenkammer zu liegen kommt.

Erfindungsgemäß ist vorgesehen, dass die Medikamentenkammer des Einzeldosis-Pulverinhalators nach Befüllung mit der Pulverdosis (und ggf. auch dem Desagglomerator) verschlossen wird, sodass der befüllte Einzeldosis-Pulverinhalator bis zur Anwendung aufbewahrt werden kann. Dieser Verschluss ist zum Öffnen vorgesehen, wenn der Pulverinhalator zur Inhalation des enthaltenen Pulvers eingesetzt werden soll. Zum Zweck des Verschlusses sind erfindungsgemäß unterschiedliche Varianten vorgesehen.

Eine aufgrund der einfachen Bedienbarkeit für den Anwender bevorzugte Variante umfasst ein Folienelement (Peeling-Folie), beispielsweise aus Aluminium oder auch Kunststoff, mit einer Verschlusslasche, die die Medikamentenkammer verschließt.

Ein solches Folienelement kann eine mit der Verschlusslasche über einen Bandabschnitt verbundene Abziehlasche aufweisen, die sich von einer Seite der Medikamentenkammer durch den gegenüberliegenden Luftein- oder Auslasskanal aus der Luftein- oder Auslassöffnung erstreckt. D. h., entweder erstreckt sich der Bandabschnitt von der Auslassseite der Medikamentenkammer durch den Lufteinlasskanal mit der Abziehlasche aus der Lufteinlassöffnung, oder bevorzugt von der Einlassseite der Medikamentenkammer durch den Auslasskanal aus der Auslassöffnung heraus, da der Kanalquerschnitt an der Auslassseite der Medikamentenkammer größer ist. Wird an der Abziehlasche Zug ausgeübt, so wird das Verschlusselement von der Medikamentenkammer abgezogen, sodass das Pulvermedikament freigesetzt wird, und kann aus dem Inhalatorgehäuse entfernt werden.

Alternativ zu der Abziehlasche kann zur Öffnung der Verschlusslasche, die die Medikamentenkammer verschließt, vorgesehen sein, dass das Deckelungselement gegenüberliegend zu der Medikamentenkammer zu einem elastischen Druckelement ausgeformt ist, das auf der Innenseite, als auf der zu der Medikamentenkammer weisenden Seite, ein oder mehrere Durchstechelemente aufweist, um die Verschlusslasche perforieren zu können, indem Druck auf das Druckelement ausgeübt wird.

Alternativ zu einem Folienelement kann die Medikamentenkammer durch zwei Stopfenelemente verschlossen sein, wobei jedes Stopfenelement einen Stopfenabschnitt aufweist, der in einem zu der Medikamentenkammer benachbarten Abschnitt des Lufteinlasskanals und des Auslasskanals angeordnet ist: Ferner weist das Stopfenelement eine Abziehlasche auf, die sich jeweils aus der Lufteinlassöffnung und der Auslassöffnung heraus erstreckt. Um den Pulverinhalator inhalationsbereit zu machen, werden hier die Stopfenabschnitte durch Zug an den Abziehlaschen aus dem Lufteinlasskanal und dem Auslasskanal entfernt.

Eine weitere, technisch allerdings aufwändigere Alternative zum Verschluss der Medikamentenkammer sieht vor, dass in dem Gehäuseteil beidseitig der Medikamentenkammer quer bzw. rechtwinklig zu dem Lufteinlasskanal und dem Auslasskanal ein seitlich begrenzter Sperrkanal ausgeformt ist, der dadurch dem Gehäuseteil erhöhte Stabilität verleiht und in dem ein korrespondierend ausgeformter Falz des Deckelungselements den Lufteinlasskanal und den Auslasskanal abdichtend verschließend aufgenommen ist. Damit diese Falze aus dem Sperrkanal angehoben werden kann, um den Lufteinlasskanal und den Auslasskanal zur Öffnung der Medikamentenkammer freizugeben, weist der Pulverinhalator ein Anheb-Element auf, für das erfindungsgemäß wiederum verschiedene Ausführungsformen vorgesehen sind.

Ein Anheb-Element eines erfindungsgemäßen Pulverinhalators kann zumindest ein vorbestimmter und markierter Druck-, Knick- oder Zugpunkt an dem Inhalatorgehäuse, d. h. dem Deckelungselement sein, sodass durch Ausüben von Druck oder Zug oder Knicken an bestimmten Stellen des Inhalatorgehäuses die Falze aus den Sperrkanälen gehoben werden.

Alternativ hierzu kann als Anheb-Element ein Zugband vorgesehen sein, das sich durch den Lufteinlasskanal und den Auslasskanal und quer durch den Sperrkanal zwischen dem Falz des Deckelungselements und dem Gehäuseteil erstreckt. Dieses Zugband kann beidenends einen Griffabschnitt aufweisen, sodass zum Anheben der Falze gleichzeitig an beiden Enden des Zugbands gezogen wird. Alternativ und aufgrund des einfacheren Handlings bevorzugt, kann das Zugband nur an einem Ende einen Griffabschnitt aufwelsen, während das andere Ende an einer Ankerstruktur des Inhalatorgehäuses verankert ist. Diese Ankerstruktur kann gleichzeitig als Luftumlenk- oder Leitstruktur im Lufteinlass- oder Auslasskanal ausgebildet sein.

Ferner können auch die oben beschriebenen Stopfenelemente als Anhebelemente verwendet werden, indem sie den Falz beim Herausziehen aus den zu der Medikamentenkammer benachbarten Kanalabschnitten anheben.

Schließlich ist auch denkbar, dass eine lose in der Medikamentenkammer angeordnete Struktur, die bevorzugt der Desagglomerator sein kann, dazu verwendet werden kann, den Falz anzuheben.

Zwar ist ein erfindungsgemäßer Pulverinhalator zum einmaligen Gebrauch konzipiert, in der bevorzugten kostengünstigen Ausführungsform mit der sortenreinen Kunststofftiefziehfolie für das Gehäuseteil und Aluminium- oder Kunststofffolie für die Deckelung eignen sich die Komponenten gut zum Recyceln. Zudem kann auch vorgesehen sein, dass der zur Herstellung verwendete Kunststoff vorzugsweise biologisch abbaubar sein kann, falls er nicht dem Recycling zugeführt wird.

Um erhöhten hygienischen Anforderungen zu entsprechen, kann der verwendete Kunststoff ein antiseptischer und/oder antimikrobieller Kunststoff sein. Alternativ hierzu kann zumindest der Lufteinlasskanal, die Medikamentenkammer und der Auslasskanal, sowie das Mund- oder Nasenstück mit einer antiseptischen und/oder antimikrobiellen Beschichtung versehen sein.

Zum Schutz vor Plagiaten kann der Kunststoff ferner einen Marker enthalten, der sich am fertigen Pulverinhalator nachweisen lässt.

Schließlich können, etwa wenn gleichzeitig zwei oder mehr Wirkstoffe verabreicht werden sollen, die Medikamentenkammer durch zumindest eine Trennwand in zumindest zwei Teilkammern unterteilt sein oder in dem Gehäuseteil zwei oder mehr Medikamentenkammern ausgeformt sein, die parallel nebeneinander mit jeweils einem Lufteinlasskanal und einem Auslasskanal oder in Serie hintereinander angeordnet sind, wobei der Lufteinlasskanal zu der ersten Medikamentenkammer führt und der Auslasskanal sich von der letzten Medikamentenkammer erstreckt und die Medikamentenkammern über einen weiteren Kanal miteinander verbunden sind. Es kann aber auch sein, dass eine Aufteilung einer größeren Einzeldosis in mehrere Medikamentenkammern strömungstechnisch vorteilhafter ist, um eine vollständige Desaggomeration und Entleerung sowie gleichmäßige Dispersion und damit Inhalation des enthaltenen Pulvers zu erreichen.

Ein erfindungsgemäßer Einzeldosis-Pulverinhalator kann also in einer einzigen Medikamentenkammer eine Gesamtdosis eines pulverförmigen Medikaments enthalten oder in zwei oder mehr Teilkammern bzw. parallelen oder seriellen Medikamentenkammern jeweils eine Teildosis enthalten, die bei einer Anwendung gleichzeitig inhaliert werden.

Sind die Teilkammern durch ein Folienelement oder die mehreren Kammern durch mehrere Folienelemente verschlossen, kann das geformte Deckelungselement zur Öffnung der Folienelemente in korrespondierender Weise mit Durchstechelementen ausgestattet sein. D. h., für den Fall, dass eine Medikamentenkammer durch eine oder mehrere Trennwände in Teilkammern unterteilt ist, sind an dem Druckelement, das gegenüber der Medikamentenkammer in dem Deckelungselement ausgebildet ist, für jede Teilkammer zumindest ein Durchstechelement vorgesehen, sodass bei Druck auf das Druckelement das Folienelement über jeder Teilkammer perforiert wird und so die darin vorliegende pulverförmige Substanz freigesetzt werden kann. Liegt ein Gehäuseteil mit mehreren Pulverkammern vor, so sind an dem geformten Deckelungselement entsprechend mehrere Druckelemente mit dem jeweiligen zumindest einen Durchstechelement vorgesehen.

Ein erfindungsgemäßes Verfahren zur Herstellung eines erfindungsgemäßen Einzeldosis-Pulverinhalators kann vorteilhaft in einer einzigen Vorrichtung ausgeführt werden. Diese ist entsprechend einer Blistermaschine zum Folientiefziehen, Abfüllen und Versiegeln ausgebildet. Das Verfahren sieht zunächst das Spritzgießen oder - bevorzugt - Tiefziehen des Gehäuseteils aus Kunststoff vor, woraufhin die Medikamentenkammer mit einer Dosis eines pulverförmigen Medikaments befüllt wird. Parallel zur Fertigung des Gehäuseteils und/oder der Befüllung wird das Deckelungselement je nach Ausführungsart einfach durch ein Trennverfahren wie Ausschneiden oder Stanzen des flächigen und im wesentlichen planaren Deckelungselement aus eine Folie oder durch Spritzgießen oder - bevorzugt - Tiefziehen aus Kunststoff gefertigt. Nach dem Verschließen der Medikamentenkammer mit einem Folienelement, wobei die Verschlusslasche an der Medikamentenkammer versiegelt oder verklebt wird, wird das Deckelungselement an dem Gehäuseteil angefügt, vorzugsweise ebenfalls durch Siegelung, Klebung oder auch durch Schweißen (z. B. Ultraschallschweißen). Beim Verschließen der Medikamentenkammer mit einem Folienelement, das zum Öffnen der Medikamentenkammer durch Abziehen der Verschlusslasche vorgesehen ist, wird vor dem Anfügen des Deckelungselements der zu der Abziehlasche führende Bandabschnitt gefaltet bzw. umgeklappt, sodass die Abziehlasche aus einer der Öffnungen des Gehäuseteils herausragt. Alternativ zu dem Folienelement kann die Medikamentenkammer gegebenenfalls auch durch zwei Stopfenelemente und/oder durch einen in einem Sperrkanal aufgenommenen Falz des Deckelungselements verschlossen werden.

Dieses einfache Verfahren ermöglicht eine äußerst kostengünstige Herstellung des Pulverinhalators und ist außerdem schnell und einfach an verschiedene Varianten anpassbar, sodass auch patientenspezifische Ausführungsformen und Befüllungen ökonomisch möglich sind. Ferner eignet sich dieser Pulverinhalator, der kostengünstige Herstellung mit optimierter Pulverfreigabe verbindet, damit auch zum Vertrieb oder Verteilung in Ländern der Dritten Welt.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich, Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine perspektivische Ansicht des Gehäuseteils eines erfindungsgemäßen Einzeldosis-Pulverinhalators,
- **Fig. 2**: eine Draufsicht auf das Gehäuseteil aus Fig. 1,
- **Flg. 3**: eine Längsschnittansicht durch das Gehäuseteil aus Fig. 1,
- **Fig. 4**: eine perspektivische Ansicht des Gehäuseteils aus Fig. 1, bei dem die Medikamentenkammer mit einem Folienelement verschlossen ist,
- **Fig. 5**: eine perspektivische Ansicht eines erfindungsgemäßen Pulverinhalators mit dem Gehäuseteil aus Fig. 4, das mit einer planen (Aluminium-)Folie als Deckelungselement verschlossen ist,
- **Fig. 6**: eine Längsschnittansicht durch den erfindungsgemäßen Pulverinhalator aus Fig. 5,
- **Fig. 7**: eine perspektivische Ansicht eines erfindungsgemäßen Pulverinhalators mit einem zur nasalen Anwendung ausgeformten Auslass,
- **Fig. 8**: eine perspektivische Ansicht eines Gehäuseteils, bei dem zum Verschluss der Medikamentenkammer zwei Stopfenelemente vorgesehen sind,
- **Fig. 9**: eine perspektivische Ansicht eines erfindungsgemäßen Pulverinhalators mit dem Gehäuseteil aus Fig. 8, das mit einer planen (Aluminium-)Folie als Deckelungselement verschlossen ist,
- **Fig. 10**: eine perspektivische Ansicht eines Gehäuseteils, bei dem zum Verschluss der Medikamentenkammer zwei Sperrkanäle und zum Öffnen ein Zugband vorgesehen sind,
- **Fig. 11**: eine perspektivische Ansicht eines erfindungsgemäßen Pulverinhalators mit dem Gehäuseteil aus Fig. 10, das mit einer planen (Aluminium-)Folie als Deckelungselement verschlossen ist, dessen Falze in den Sperrkanälen aufgenommen sind,
- **Fig. 12**: eine schematische Seitenschnittansicht eines erfindungsgemäßen Pulverinhalators, bei dem die Medikamentenkammer durch Falze im Deckelungselement in den Sperrkanälen verschlossen ist, die durch Zug an dem Deckelungselement angehoben werden,
- **Fig. 13**: eine schematische Seitenschnittansicht eines erfindungsgemäßen Pulverinhalators, bei dem die Medikamentenkammer durch Falze im Deckelungselement in den Sperrkanälen verschlossen ist, die durch Druck an dem Deckelungselement und Knickelemente angehoben werden,
- **Fig. 14**: eine Längsschnittansicht durch einen erfindungsgemäßen Pulverinhalator mit geformtem Deckelelement, das ein Druckelement mit Durchstechelement zur Öffnung der Verschlusslasche aufweist,
- **Fig. 15**: eine Längsschnittansicht durch einen weiteren erfindungsgemäßen Pulverinhalator mit geformtem Deckelelement, bei dem das Druckelement zwei Durchstechelemente zur Öffnung der Verschlusslasche über den zwei Teilkammern aufweist,
- **Fig. 16**: eine perspektivische Ansicht auf eine Tiefziehfolie mit vier ausgeformten Gehäuseteilen, an die Deckelungselemente angefügt sind, vor Ausschneiden der Pulverinhalatoren,
- **Fig. 17**: eine schematische Darstellung einer Fertigungsvorrichtung zur Herstellung erfindungsgemäßer Pulverinhalatoren.

Die erfindungsgemäße Vorrichtung bezieht sich auf einen einfach und kostengünstig herstellbaren Pulverinhalator aus zwei Gehäuseteilen, einem geformten Gehäuseteil und einem Deckelungselement, die zusammengefügt das Inhalatorgehäuse bilden. Der erfindungsgemäße Pulverinhalator kann in der Art einer Sichtverpackung bzw. blisterartig ausgeführt sein, wobei das Gehäuseteil oder das Deckelungselement oder beide transparent sind und so einen Einblick in den Pulverinhalator, insbesondere in die Medikamentenkammer gestattet/n. Mit dem zumindest teilweise transparent gestalteten Inhalator kann der Inhalt bzw, die vollständige Entleerung des Inhalts in der Anwendung kontrolliert werden. Auch kann die Verwirbelung während der Anwendung verfolgt werden.

Das Gehäuseteil ist zur Bereitstellung des Lufteinlasskanals, der Medikamentenkammer und des Auslasskanals ausgeformt und kann vorteilhaft kostengünstig durch eine Kunststofftiefziehfolie gefertigt werden oder gegebenenfalls auch im Kunststoffspritzguss hergestellt werden.

Zur Begrenzung des Lufteinlasskanals, der Medikamentenkammer und des Auslasskanals kann einfach eine pharmagerechte Folie, die eine Aluminium-, Verbund- oder (transparente) Kunststofffolie sein kann, als Deckelungselement an dem Gehäuseteil angefügt werden, beispielsweise durch Siegelung. Schweißen oder Kleben.

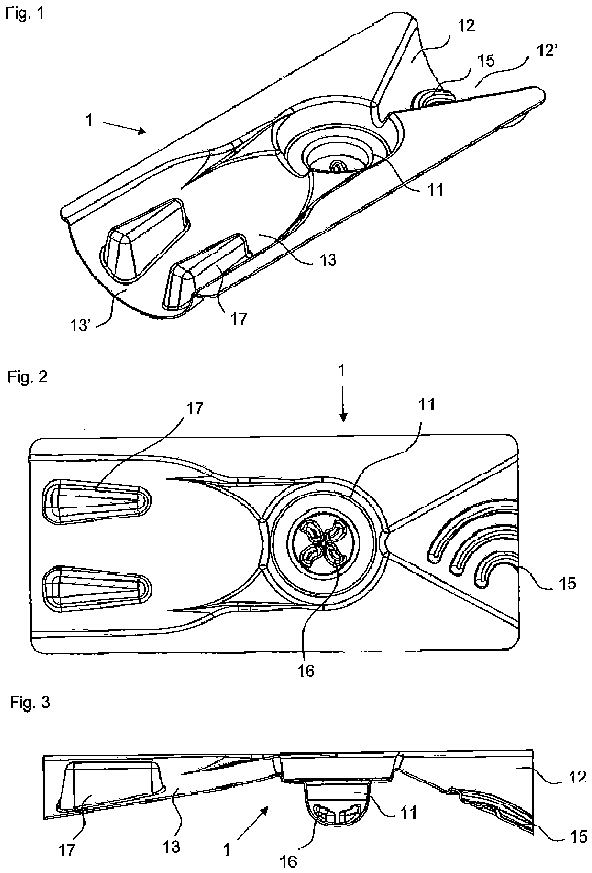

Figuren 1 bis 3 zeigen ein Beispiel für ein Gehäuseteil 1, wie es zur Herstellung eines erfindungsgemäßen Pulverinhalators eingesetzt wird. Das hier aus Tiefziehfolie geformte Gehäuseteil 1 weist einen halbtrichterförmigen Lufteinlasskanal 12 auf, der sich von einer Lufteinlassöffnung 12' zu der schalenartig ausgeformten Medikamentenkammer 11 erstreckt. Auf der von dem Lufteinlass abgewandten Seite erstreckt sich der Auslasskanal 13 von der Medikamentenkammer 11 zur Auslassöffnung 13', die hier einen halbovalen Querschnitt aufweist, um gut im Mund aufgenommen werden zu können.

In dem Lufteinlasskanal 12 sind mehrere Luftleitstrukturen 15 ausgeformt, um den in der Anwendung eingesogenen Luftstrom in Torsion zu versetzen, ehe er in die Medikamentenkammer 11 gelangt. Im Boden der Medikamentenkammer 11 sind Luftverwirbelungsstrukturen 16 ausgeformt, die die Desagglomeration des aufgewirbelten Pulvers verbessern. Eine Aufweitung des Auslasskanals 13 im Anschluss an die Medikamentenkammer 11 sorgt für einen Spacer-Effekt, wobei nicht nur die zuvor durch den trichterförmigen Einlass erhöhte Strömungsgeschwindigkeit verlangsamt wird, sondern auch das mitgerissene Pulver gleichmäßig verteilt wird. Dieser Effekt wird durch die Leitstrukturen 17 unterstützt, die zudem dem Gehäuseteil 1 in dem Auslassbereich eine erhöhte Stabilität verleihen.

Nach der Herstellung der Gehäuseteils 1, was vorzugsweise durch Folientiefziehen erfolgt, gegebenenfalls aber auch im Spritzguss erfolgen kann, wird die Medikamentenkammer 11 mit einer Pulverdosis (gegebenenfalls auch noch einem losen Desagglomerator) befüllt (nicht in den Figuren dargestellt) und anschließend verschlossen.

Der Verschluss der Medikamentenkammer 11 kann unterschiedlich erfolgen.

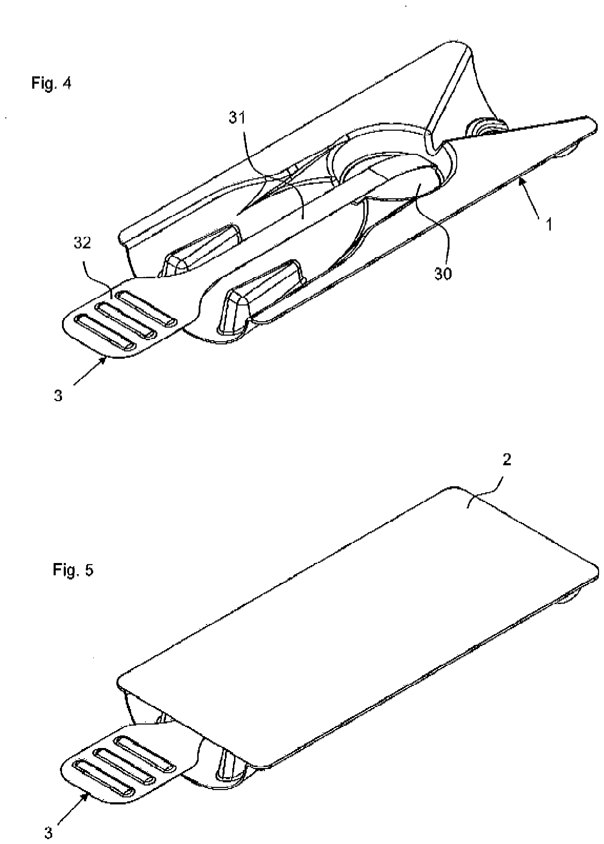

Figuren 4 bis 6 zeigen eine Ausführungsform, in der zum Verschluss der Medikamentenkammer 11 ein Folienelement 3 eingesetzt wird. Das Folienelement 3 hat eine Verschlusslasche 30, die so dimensioniert ist, dass sie die Medikamentenkammer 11 verschließt. Von dieser Verschlusslasche 30 erstreckt sich ein Bandabschnitt 31 zu einer Abziehlasche 32, die hier für sichereres Greifen geriffelt ist. Dabei ist der Bandabschnitt 31 an der Verschlusslasche 30 umgeklappt, sodass die Abziehlasche 32 auf der von dem umgeklappten Ende abgewandten Seite aus dem Gehäuseteil 1 herausragt. In dem gezeigten Beispiel ist der Bandabschnitt 31 an der Einfass-Seite der Verschlusslasche 30 umgelenkt und ragt mit der Abziehlasche 32 aus der Auslassöffnung 13' heraus.

Generell ist auch denkbar, dass ein solches Folienelement 3 auch umgekehrt angeordnet sein kann, sodass die Abziehlasche 32 aus der Einlassöffnung 12' herausragt; da allerdings der Querschnitt des Auslasskanals 13 an der Medikamentenkammer 11 vorzugsweise größer ist als der Querschnitt des Einlasskanals 12, bietet sich der Auslasskanal 13 zum Abziehen der Verschlusslasche 30 an. Die Verschlusslasche 30 kann mit einem Wandabschnitt bzw. einem umlaufenden Absatz der Medikamentenkammer 11 versiegelt, verschweißt oder verklebt werden, wobei die Verbindung zwar dicht aber doch lösbar ausgeführt ist, sodass sich die Verschlusslasche 30 durch Zug an der Abziehlasche 32 lösen lässt, ohne zu zerreisen.

Das in **Figuren 1 bis 6** dargestellte Beispiel stellt eine bevorzugte Variante eines erfindungsgemäßen Pulverinhalators dar, der aufgrund des blisterartigen Aufbaus äußerst einfach und kostengünstig in einer einzigen Maschine hergestellt werden kann und zudem für die Anwender einfach und intuitiv zu bedienen ist.

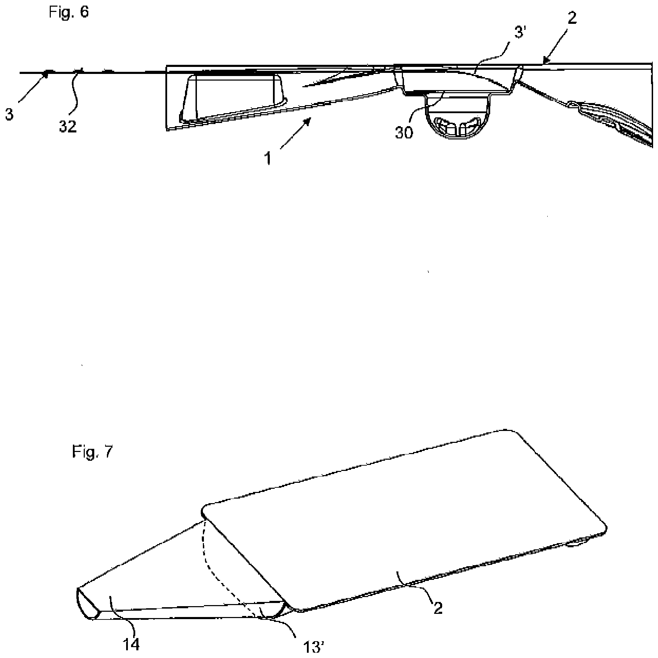

In **Flg. 7** ist beispielhaft dargestellt, dass ein erfindungsgemäßer Pulverinhalator nicht nur mit einem Auslass ausgeformt sein kann, der zur Aufnahme in den Mund geformt ist, An der (gedachten und gestrichelt eingezeichneten) Auslassöffnung 13' kann sich ein zur nasalen Anwendung ausgeformtes Auslassstück 14 anschließen, das sich einstückig von dem Gehäuseteil wegerstrecken kann, oder das auf die Auslassöffnung 13' aufgesetzt werden kann.

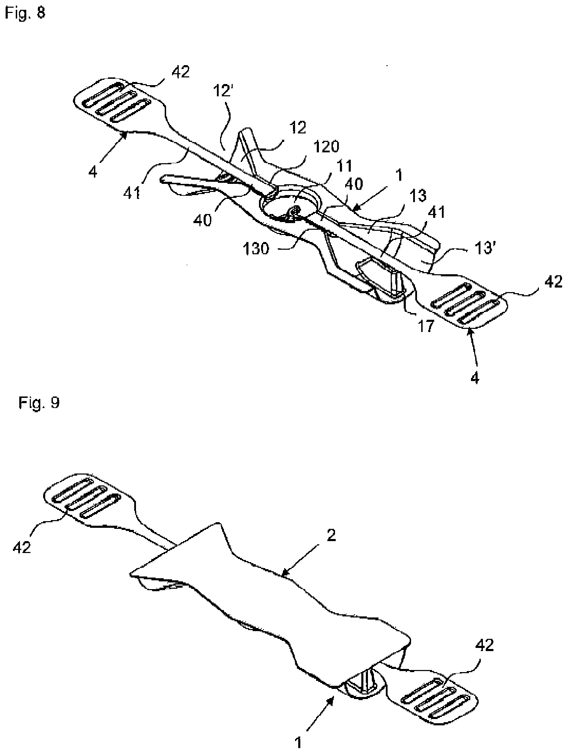

**Figuren 8 und 9** zeigen eine Variante eines erfindungsgemäßen Pulverinhalators, bei dem der Verschluss der Medikamentenkammer 11 durch zwei Stopfenelemente 4 erfolgt, ehe das Deckelungselement 2 den Lufteinlasskanal 12, die Medikamentenkammer 11 und den Auslasskanal 13 deckelt. Die Stopfenelemente 4 haben jeweils einen Stopfenabschnitt 40, von dem sich ein Bandabschnitt 41 zu einer Abziehlasche 42 erstreckt. Der Stopfenabschnitt 40 des jeweiligen Stopfenelements 4 wird in einem an die Medikamentenkammer 11 angrenzenden Abschnitt 120, 130 des Lufteinlasskanals 12 und des Auslasskanals 13 angeordnet und ist so dimensioniert und geformt, dass er diesen Abschnitt 120,130 verschließt, Ein Material der Stopfenabschnitte 40 wird so gewählt, dass ein dichter Verschluss ermöglicht wird, dieser aber durch Zug an den Abziehlaschen 42 überwunden werden kann. Alternativ kann zwischen Stopfenabschnitt 40 und Kanalabschnitt 120,130 ein Kleb- oder Dichtmittel eingebracht sein, das die Entfernung der Stopfenabschnitte 40 bei Zug an den Abziehlaschen 42 zerstörungsfrei erlaubt.

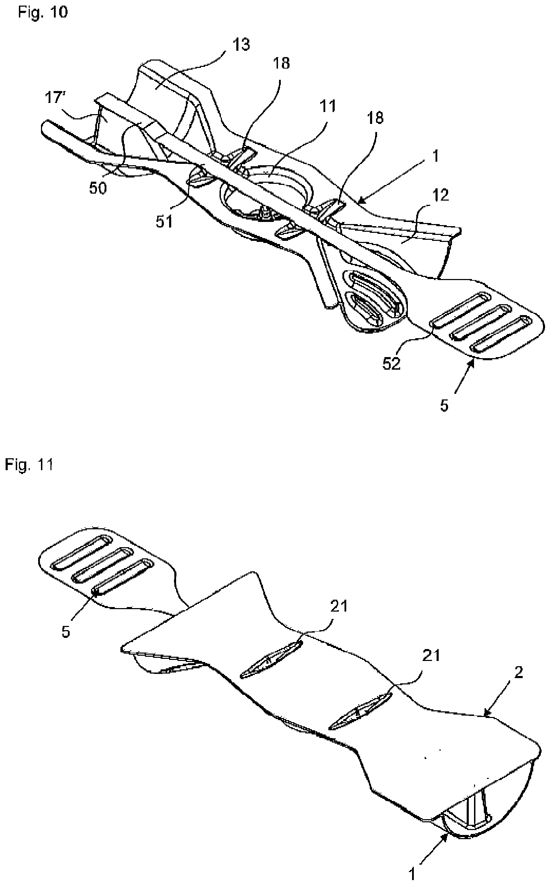

Eine weitere Alternative zum Verschluss der Medikamentenkammer ist in **Figuren 10 bis** 13 gezeigt. In **Figuren 10 und 11** ist ein Gehäuseteil 1 zu sehen, bei dem in den zu der Medikamentenkammer 11 benachbarten Abschnitten 120, 130 des Lufteinlasskanals 12 und des Auslasskanals 13 ein quer dazu verlaufender Sperrkanal 18 ausgeformt ist. Das im Wesentlichen planare Deckelungselement 2 weist an den diesen Sperrkanälen 18 entsprechenden Stellen eingeformte Falze 21 auf, die die Sperrkanäle 18 ausfüllen und damit die Abschnitte 120, 130 des Lufteinlasskanals 12 und des Auslasskanals 13 sperren und so die Medikamentenkammer 11 verschließen. Um eine derart verschlossene Medikamentenkammer 11 zu öffnen und den Lufteinlasskanal 12 und den Auslasskanal 13 freizugeben, werden die Falze 21 aus den Sperrkanälen 18 angehoben, wozu verschiedene erfindungsgemäße Varianten vorgesehen sind.

In dem in **Figuren 10 und 11** gezeigten Beispiel ist hierzu ein Zugband 5 vorgesehen, dessen Bandabschnitt 51 sich von dem Auslasskanal 13 quer durch die Sperrkanäle 18 und die Medikamentenkammer 11 bis zu dem Lufteinlasskanal 12 erstreckt. Das Zugband 5 ist mit einem Endabschnitt 50 an einer Ankerstruktur 17' im Auslasskanal 13 befestigt, die gleichzeitig als Luftleitstruktur dient. Am anderen Ende des Zugbands 5 ragt eine Lasche 52 aus dem Lufteinlasskanal 12 heraus. Der Bandabschnitt 51 wird bei Anordnung des Deckelungselements 2 mit den Falzen 21 in die Sperrkanäle 18 gedrückt. Zur Freigabe des Lufteinlasskanals 12 und des Auslasskanals 13 wird Zug an der Lasche 52 auf den Bandabschnitt 51 ausgeübt, der an der Ankerstruktur 17' gehalten wird, sodass der Bandabschnitt 51 die Falze 21 aus den Sperrkanälen 18 anheben kann. Anders als dargestellt, kann ein solches Zugband auch unverankert mit zwei Laschen ausgeführt sein, sodass zum Anheben der Falze 21 und zur Freigabe des Lufteinlasskanals 12 und des Auslasskanals 13 an beiden Laschen gezogen wird.

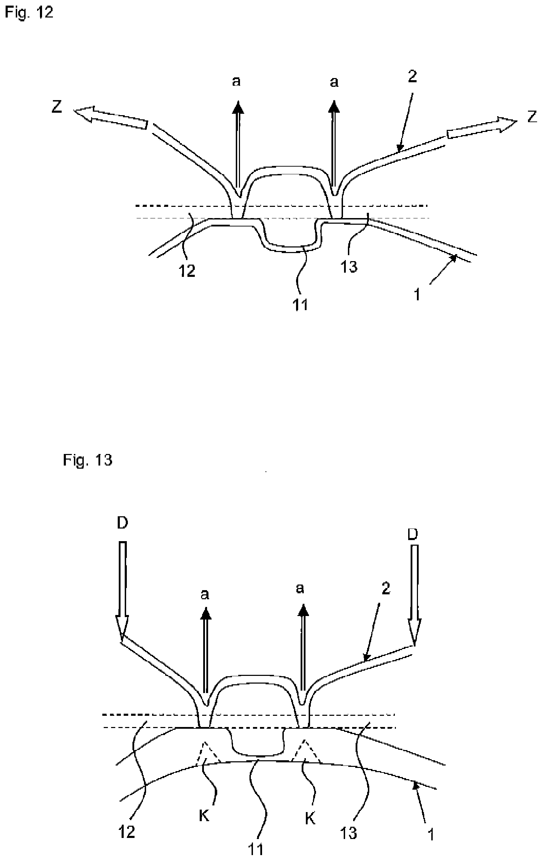

Alternativ dazu kann, wie in **Fig. 12** angedeutet ist, die Zugkraft Z an dem Deckelungselement 2 ausgeübt werden, um die Falze anzuheben, wie die Doppelpfeile a andeuten. Es kann aber auch vorgesehen sein, dass, wie in **Fig. 13** angedeutet, an bestimmten Stellen Druck D auf das Deckelungselement 2 ausgeübt wird, um die Falze anzuheben und/oder das Gehäuse an dafür vorgesehenen Stellen K zu knicken.

Eine weitere Möglichkeit zur Freigabe des Strömungskanals durch Lufteinlasskanal 12 und Auslasskanal 13 und Öffnung der Medikamentenkammer 11 besteht im Lösen von Stopfenelementen (wie oben beschrieben) zur Anhebung der Falze. Ferner kann eine lose in der Medikamentenkammer angeordnete, und damit frei bewegliche, vorzugsweise kugelige Struktur, die in der Anwendung auch als Desagglomerator fungiert, durch Anheben dazu verwendet werden, die Falze in den Sperrkanälen anzuheben.

Generell kann ein erfindungsgemäßer Pulverinhalator von den gezeigten Ausführungsbeispielen abweichen, so ist die Erfindung nicht auf die in den Figuren gezeigten Anzahlen und Formen der Luftverwirbelungs-, Umlenk- und Leitstrukturen 15,16,17 bzw. 17' beschränkt; die im Lufteinlasskanal 12, in der Medikamentenkammer 11 und dem Auslass 13 zur Verbesserung der Aerodynamik, des Widerstandes und/oder der Desagglomeration ausgeformten Strukturen der inneren Wandung eines erfindungsgemäßen Einzeldosis-Pulverinhalators können als intraluminale Strömungselemente von den dargestellten Formen abweichen und beispielsweise als Schikanen, Schaufeln, Schnecken, Spiralen oder Mäander geformt sein.

Weiter ist das Strömungsprofil durch die Medikamentenkammer aerodynamisch auf Widerstand, Durchfluss und Pulver durch Variationen des Bohrungsprofils hinsichtlich Größe, Form und Kantengestaltung (scharfkantig, angefast oder abgerundet) am Ein- und Auslass der Medikamentenkammer abstimmbar. So beeinflussen verschiedene Faktoren die Luftströmung in Geschwindigkeit und Verlauf durch den Pulverinhalator und insbesondere die Medikamentenkammer, die Einfluss auf die Vollständigkeit der Entleerung und Desagglomeration und Dispersion im Luftstrom haben, wie zum Beispiel der verjüngende Querschnittsverlauf in Lufteinlass und der aufweitende Querschnitt im Auslass, wobei der Strömungskanal auf Einlassseite der Medikamentenkammer vorzugsweise enger ist als auf der Auslassseite.

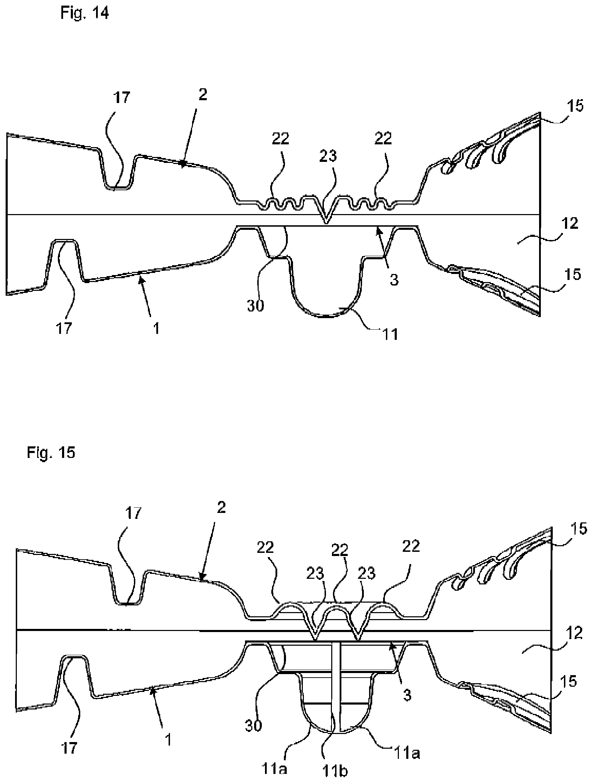

**Fig. 14** und **Fig. 15** zeigen erfindungsgemäße Pulverinhalatoren, bei denen nicht nur das Gehäuseteil 1 mit der Medikamentenkammer 11, sondern auch das Deckelungselement 2 geformt ist. In dieser Ausführungsform korrespondieren die jeweiligen Abschnitte zur Bildung des Lufteinlasskanals 12 und des Auslasskanals 13 an Gehäuseteil 1 und Deckelungselement 2 miteinander sowohl in Öffnungswinkel als auch in den ausgeformten Luftverwirbelungs-, Umlenk- und Leitstrukturen 16, 17. Das Deckelungselement 2 unterscheidet sich lediglich in dem zwischen Ein- und Auslasskanal 12,13 liegenden Abschnitt von dem Gehäuseteil 1: Anstelle der Medikamentenkammer 11 weist das Deckelungselement 2 einen als elastischen Druckelement 22 ausgebildeten Bereich, der in **Fig. 14** mittig ein nach Innen in Richtung der Medikamentenkammer 11 weisendes Durchstechelement 23 und in **Fig. 15** zwei Durchstechelemente 23 aufweist, die jeweils den beiden Teilkammern 11a gegenüberliegen, die in der Medikamentenkammer 11 durch die Trennwand 11b ausgebildet sind.

Das Druckelement 22 wird durch konzentrische Ringwallstrukturen gebildet, die eine elastische Verformung der Kunststofffolie an dieser Stelle zulassen, sodass das nach innen angeformte Durchstechelement 23 durch Druckausübung in Richtung der Medikamentenkammer 11 zum Durchstechen der Verschlusslasche 30 bewegt werden kann und nach Lösen des Drucks in seine Ausgangslage zurückkehrt. Das Durchstechelement kann aus einer oder mehreren Nadeln bestehen oder durch eine kantige Struktur wie ein pyramidenförmiger Dorn gebildet sein. Wird zum Öffnen beidseitiger Druck, d. h. Druck auf das Druckelement 22 und Gegendruck auf die Medikamentenkammer 11, ausgeübt, so wird die Verschlusslasche 30, die beispielsweise aus Aluminiumfolie bestehen kann, richtiggehend aufgesprengt, da durch den Gegendruck auf die Medikamentenkammer 11 die Verschlusslasche 30 unter Spannung gesetzt wird und dem Durchstechelement 23 nicht ausweichen kann.

Das Gehäuseteil und das Deckelungselement eines erfindungsgemäßen Einzeldosis-Pulverinhalators können durch Wärmeeinwirkung miteinander verschweißt werden, es kommen aber auch Verkleben, Klemmverbindungen (wobei der Rand eines Gehäuseteils um den Rand des anderen Gehäuseteils herumgebogen wird), Rastverbindungen oder Heftverbindungen in Frage.

Der erfindungsgemäße Pulverinhalator ist zur einmaligen Verwendung vorgesehen, sodass das Inhalatorgehäuse nach dem Öffnen der Medikamentenkammer und der Inhalation der Pulverdosis nicht wieder verwendet werden kann, was auch dem Schutz vor Fehlanwendungen dient. Neben der guten Recyclebarkeit oder der Bloabbaubarkeit relativiert sich das aus Sicht des Umweltschutzes als nachteilig zu betrachtende erhöhte Verpackungsmüllvolumen aber auch durch den verringerten Einsatz von Multidose-Inhalatoren, deren Entsorgung aufwändiger ist, da meist Restmengen des pulverförmigen Medikaments enthalten sind.

Der erfindungsgemäße Einzeldosis-Pulverinhalator ermöglicht die wirtschaftliche Anwendung für diverse Indikationen - etwa, wenn nur wenige Anwendungen oder nur bei Bedarf erforderlich sind, wie z. B. bei Migräne, Schmerz, Immunmodulatoren, Biologicals oder die Verabreichung ZNS-wirksamer Substanzen. Der erfindungsgemäße Pulverinhalator ist hygienisch und unerwünschte Einflüsse wie hohe Luftfeuchtigkeit oder Verschmutzung durch falsche Benutzung sind weitestgehend ausgeschlossen.

Wegen der kostengünstigen Herstellung eignet sich der erfindungsgemäße Pulverinhalator auch zum Einsatz in der Dritten Welt und in Krisengebieten.

Tatsächlich ist der erfindungsgemäße Pulverinhalator so günstig in der Herstellung, dass sich eine patientenindividuelle Arzneimittelverblisterung in ökonomisch vertretbarem Rahmen realisieren lässt. Die Zusammensetzung und Dosierung des pulverförmigen Medikaments (bzw. mehrere Medikamente) lassen sich somit für jeden Patienten individuell und maßgeschneidert in dem erfindungsgemäßen Pulverinhalator abfüllen.

Im Zusammenhang mit den Figuren 1 bis 14 sind Ausführungsbeispiele eines erfindungsgemäßen Einzeldosis-Pulverinhalators mit einer einzigen Medikamentenkammer, in der eine Pulverdosis vorliegt, gezeigt. **Fig. 15** zeigt eine erfindungsgemäße Variante, in der die Medikamentenkammer 11 durch die hier einstückig mit dem Gehäuseteil 1 ausgeformte Trennwand 11b in zwei Teilkammern 11a unterteilt ist, sodass zwei verschiedene pulverförmige Substanzen bzw. Medikamente, die getrennt voneinander aufbewahrt werden sollen, erst nach Öffnen der Verschlusslasche beim Inhalieren zusammen kommen.

Es ist aber erfindungsgemäß auch denkbar, dass zwei oder mehr Medikamentenkammern in dem Gehäuseteil ausgeformt sind, die parallel oder in Serie im Strömungskanal zwischen Lufteinlass und Auslass angeordnet sind. Parallel bedeutet hier, dass jeweils zumindest ein Lufteinlasskanal (von einer gemeinsamen Lufteinlassöffnung oder getrennten Lufteinlassöffnungen) zu jeder der Medikamentenkammern führt und jeweils zumindest ein Auslasskanal aus jeder Medikamentenkammer in den als Mundstück oder Nasenrüssel ausgeformten Auslass mündet. Bei der Anordnung der Medikamentenkammern in Serie erstreckt sich der Lufteinlasskanal zu einer ersten Medikamentenkammer, von dort ein weiterer Kanal zu der nächsten Medikamentenkammer, und der Auslasskanal von der letzten Medikamentenkammer zu der Auslassöffnung. In jeder Medikamentenkammer kann eine Pulverdosis vorliegen, wobei es sich um unterschiedliche Wirkstoffe handeln kann, die erst in der Anwendung miteinander in Kontakt kommen. So enthält ein solcher Pulverinhalator im Prinzip zwar mehr als eine Pulverdosis, erfindungsgemäß soll dieser Pulverinhalator aber dennoch als "Einzeldosis"-Pulverinhalator verstanden werden, da diese mehreren Pulverdosen kombiniert in einer Einzelanwendung inhaliert werden, woraufhin der Pulverinhalator nicht mehr verwendet werden kann.

Der Kunststoff, der zur Herstellung eines erfindungsgemäßen Pulverinhalators, der als Wegwerfartikel konzipiert ist, verwendet wird, kann vorzugsweise ein biologisch abbaubarer Kunststoff sein.

Jeder erfindungsgemäße Pulverinhalator kann zumindest teilweise mit einer antiseptischen bzw. antibakteriellen und/oder antimikrobiellen Beschichtung versehen sein. So können besonders Lufteinlasskanal, Medikamentenkammer und Luftauslasskanal beschichtet sein, um in der Anwendung der Inhalationsvorrichtung keine Keime zu inhalieren. Wegen der einfacheren Applikation kann aber auch die gesamte Inhalationsvorrichtung beschichtet sein. Ein Beispiel für eine solche Beschichtung ist Perlazid ® von Rilit, Endingen. Alternativ kann zur Herstellung der Inhalationsvorrichtung ein antiseptischer bzw. antibakterieller und/oder antimikrobieller Kunststoff verwendet werden.

Ein erfindungsgemäßer Pulverinhalator kann beispielsweise auch in einer Umverpackung vertrieben werden, die sicherstellt, dass der Pulverinhalator sauber bzw. steril und damit sofort anwendungsbereit ist. Alternativ ist eventuell denkbar, dass das Deckelungselement an den Enden einen Überlapp aufweist, mit dem der Auslass (gegebenenfalls auch der Lufteinlass) nach Art eines Joghurtbecherdeckels verschlossen werden kann. Der Überlapp kann dann beispielsweise mittels einer ausgeformten Lasche von der oder den Öffnungen abgezogen werden. Um zu verhindern, dass die Folie dann auch von dem Gehäuseteil abgezogen werden kann, kann an der Verbindungsstelle des Überlapps eine Sollbruchstelle, beispielsweise eine Perforation vorgesehen sein.

Ferner kann der zur Herstellung eines erfindungsgemäßen Pulverinhalators eingesetzte Kunststoff einen Marker zu seiner Erkennbarkeit aufweisen, um Plaglate, die den Marker nicht enthalten, erkennen zu können.

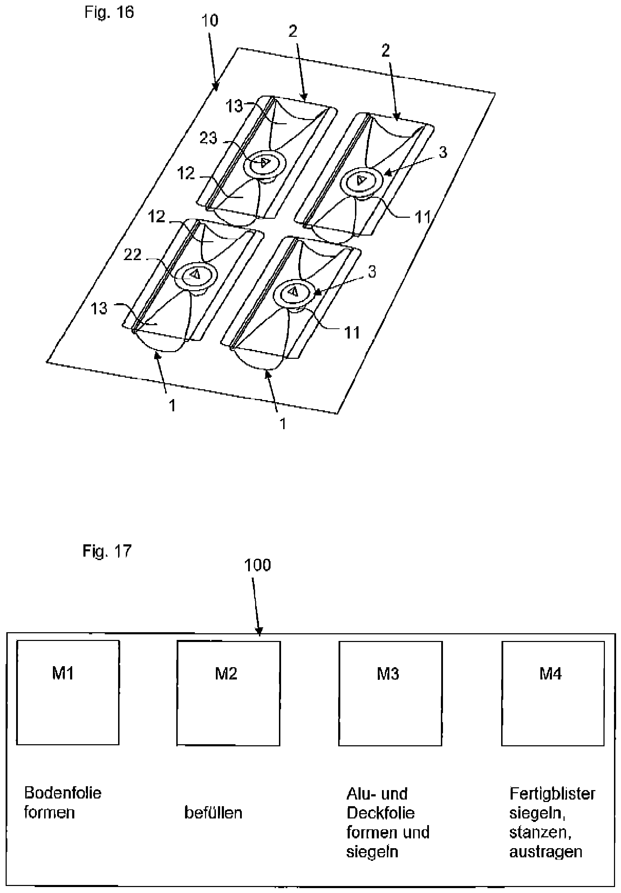

In **Fig. 16** ist eine Kunststofftiefziehfolie 10 zu sehen, in der vier Gehäuseteile 1, jeweils mit Einlass- und Auslasskanal 12,13 und Medikamentenkammer 11 ausgeformt sind. Zur besseren Übersichtlichkeit sind nicht alle Elemente der Gehäuseteile mit Bezugszeichen versehen. Die Medikamentenkammern 11 der Gehäuseteile 1 sind befüllt und mit einer Verschlusslasche 30 eines Folienelements 3 verschlossen. Ferner ist zu sehen, dass an jedem Gehäuseteil 1 jeweils ein flächiges Deckelelement 2 angefügt ist, das gegenüberliegend der Medikamentenkammer 11 ein Druckelement 22 mit Durchstechelement 23 aufweist. Zur Fertigstellung der jeweiligen Pulverinhalatoren müssen nur noch die entsprechenden befüllten Blisterstrukturen abgetrennt werden.

Anders als bei herkömmlichen Pulverinhalatoren wird somit nicht die Pulverdosis in einer Kapsel oder einem Blister bereitgestellt, die/der in ein Inhalatorgehäuse eingelegt werden muss, ehe das Pulver inhaliert werden kann. Ein erfindungsgemäßer Pulverinhalator wird quasi durch den Blister selbst, der das zu inhalierende Pulver enthält, gebildet. Das Einlegen einer pulverhaltigen Kapsel in ein Inhalatorgehäuse kann somit entfallen.

**Fig. 17** fasst die Module einer beispielhaften Fertigungsvorrichtung 100 für erfindungsgemäße Pulverinhalatoren zusammen. Selbstverständlich sind andere Fertigungsvorrichtungen zur Durchführung der erfindungsgemäßen Verfahrensschritte ebenfalls denkbar.

In dem ersten Modul M1 erfolgt das Formen des Gehäuseteils 1. In einer beispielhaften Verfahrensvariante kann dort die Tiefziehfolie für einen kontinuierlichen Prozess von Rollenware zu einer Endlosfolie verspleißt werden, ehe vorgeheizt und zur Formstation weitertransportiert wird. Alternativ dazu sind auch semikontinuierliche oder diskontinuierliche Prozesse denkbar, bei denen z.B. das Verspleißen entfallen kann. Das zweite Modul M2 ist die Befüllstation, In dem die ausgeformten Medikamentenkammern mit einer vorbestimmten Pulverart bzw. Mischung und Menge befüllt werden. Gegebenenfalls können auch für einen jeweiligen Patienten maßgeschneiderte Pulverzusammensetzungen und Dosierungen abgefüllt werden. Im dritten Modul M3 werden die Medikamentenkammern durch eine Verschlussfolie versiegelt - oder mit den Stopfenelementen verschlossen - und die Deckelelemente in einem kontinuierlichen Prozess angefügt. Im Falle geformter Deckelelemente können diese entsprechend den Gehäuseteilen in Modul 1 ebenfalls aus Tiefziehfolie nach Vorheizen geformt, auf die Tiefziehfolie mit den Gehäuseteilen aufgelegt und versiegelt werden. Im letzten Modul M4 kann eine weitere Siegelstation folgen, ehe die Pulverinhalatoren aus den Tiefziehfolien abgetrennt, vorzugsweise gestanzt, werden und als Fertigblister ausgetragen werden.

### BEZUGSZEICHENLISTE

- 1: Gehäuseteil
- 10: Tiefziehfolie
- 11: Medikamentenkammer
- 11a, 11b: Teilkammer, Trennwand
- 12, 120: Lufteinlasskanal, Abschnitt benachbart zu der Medikamentenkammer
- 12': Lufteinlassöffnung
- 13, 130: Auslasskanal, Abschnitt benachbart zu der Medikamentenkammer
- 13': Auslassöffnung
- 14: Nasenstück
- 15, 16, 17: Luftverwirbelungs-, Umlenk-, Leitstrukturen
- 17': Ankervorrichtung
- 18: Sperrkanal
- 2: Deckelungselement
- 21: Falz
- 22: Druckelement
- 23: Durchstechelement
- 3: Folienelement
- 30: Verschlusslasche
- 31: Bandabschnitt
- 32: Abziehlasche
- 4: Stopfenelement
- 40: Stopfenabschnitt
- 41: Bandabschnitt
- 42: Abziehlasche
- 5: Zugband
- 50: Endabschnitt
- 51: Bandabschnitt
- 52: Zuglasche
- 100: Fertigungsvorrichtung
- Z: Zugkraft
- D: Druck
- KD: Stellen

## Patentansprüche

1. Einzeldosis-Pulverinhalator aus einem Inhalatorgehäuse, das ein Gehäuseteil (1) aufweist, in dem zumindest eine Medikamentenkammer (11) mit einer Dosis eines pulverfömigen Medikaments ausgeformt ist, und wobei das Inhalatorgehäuse eine Auslassöffnung (13') und einen Auslasskanal (13) aufweist, der sich von der Medikamentenkammer (11) zu der Auslassöffnung (13') erstreckt,
**dadurch gekennzeichnet, dass**
das Inhalatorgehäuse zweiteilig ist und ein Gehäuseteil (1) und ein Deckelungselement (2) aufweist, das mit dem Gehäuseteil (1) zusammengefügt das Inhalatorgehäuse bildet, wobei
der Auslasskanal (13) in dem Gehäuseteil (1) ausgeformt ist,
und dass
in dem Gehäuseteil (1)
- eine Lufteinlassöffnung (12') auf einer von der Auslassöffnung (13') abgewandten Seite der Medikamentenkammer (11) und
- ein Einlasskanal (12), der sich von der Lufteinlassöffnung (12') zu der Medikamentenkammer (11) erstreckt, wobei der Auslasskanal (13), die Medikamentenkammer (11) und der Einlasskanal (12) eine Achse definieren,
ausgeformt sind,
wobei in dem Lufteinlasskanal (12), in der Medikamentenkammer (11) und in dem Auslasskanal (13) Luftleit-, Verwirbelungs- und/oder Umlenkstrukturen (15,16,17) ausgeformt sind, und
sich der Einlasskanal (12) von der Lufteinlassöffnung (12') zu der Medikamentenkammer (11) in zwei Richtungen senkrecht zu der durch den Auslasskanal (13), die Medikamentenkammer (11) und den Einlasskanal (12) definierten Achse verjüngt und sich der Auslasskanal (13) von der Medikamentenkammer (11) in Richtung der Auslassöffnung (13') in zwei Richtungen senkrecht zu der durch den Auslasskanal (13), die Medikamentenkammer (11) und den Einlasskanal (12) definierten Achse aufweitet.

2. Einzeldosis-Pulverinhalator nach Anspruch 1,
**dadurch gekennzeichnet, dass**
das Gehäuseteil (1) ein Kunststoffspritzgussteil ist oder vorzugsweise aus einer pharmagerechten, bevorzugt sortenreinen Kunststofffolie geformt, besonders bevorzugt tiefgezogen ist, wobei das Deckelungselement (2)
- flächig und im Wesentlichen planar ausgebildet ist und den Einlasskanal (12), die Medikamentenkammer (11) und den Auslasskanal (13) begrenzt, oder
- zumindest im Bereich des Einlasskanals (12) und des Auslasskanals (13) korrespondierend zu dem Gehäuseteil (1) ausgeformt ist,
und aus einer Kunststoff-, Aluminium- oder Verbundfolie besteht,
und/oder
zumindest eine Inhalatorgehäusekomponente Gehäuseteil (1) und/oder Deckelungselement (2) transparent ist.

3. Einzeldosis-Pulverinhalator nach Anspruch 1 oder 2
**dadurch gekennzeichnet, dass**
die Auslassöffnung (13') als Mundrohr ausgeformt ist oder mit einem Nasenrüssel (14) verbunden ist,
und/oder
ein Querschnitt des Lufteinlasskanals (12) an der Medikamentenkammer (11) kleiner ist als ein Querschnitt des Auslasskanals (13) an der Medikamentenkammer (11).

4. Einzeldosis-Pulverinhalator nach zumindest einem der Ansprüche 1 bis 3,
**dadurch gekennzeichnet, dass**
in der Medikamentenkammer (11) zumindest ein loser Desagglomerator angeordnet ist.

5. Einzeldosis-Pulverinhalator nach zumindest einem der Ansprüche 1 bis 4,
**dadurch gekennzeichnet, dass**
ein Folienelement (3) mit einer Verschlusslasche (30) die Medikamentenkammer (11) verschließt.

6. Einzeldosis-Pulverinhalator nach Anspruch 5,
**dadurch gekennzelchnet, dass**
das Folienelement (3) zumindest eine Abziehlasche (32) aufweist, die sich ausgehend von der Verschlusslasche (30) von einer Seite der Medikamentenkammer (11) durch den gegenüberliegenden Lufteinlasskanal oder Auslasskanal (12,13) aus der Lufteinlassöffnung oder Auslassöffnung (12',13') heraus erstreckt, oder
das Deckelungselement (2) gegenüberliegend zu der Medikamentenkammer (11) zu einem elastischen Druckelement (22) mit zumindest einem Durchstechetement (23) zur Perforation der Verschlusslasche (30) bei Ausüben von Druck auf das Druckelement (22) ausgeformt ist.

7. Einzeldosis-Pulverinhalator nach zumindest einem der Ansprüche 1 bis 4,
**dadurch gekennzeichnet, dass**
- die Medikamentenkammer (11) durch zwei Stopfenelemente (4) verschlossen ist, wobei jedes Stopfenelement (4) einen Stopfenabschnitt (40), der in einem zu der Medikamentenkammer (11) benachbarten Abschnitt (120,130) des Lufteinlasskanals (12) und des Auslasskanals (13) angeordnet ist, und eine Abziehlasche (42) aufweist, die sich jeweils aus der Lufteinlassöffnung (12') und der Auslassöffnung (13') heraus erstreckt,
oder
- in dem Gehäuseteil (1) beidseitig der Medikamentenkammer (11) rechtwinklig zu dem Lufteinlasskanal (12) und dem Auslasskanal (13) ein Sperrkanal (18) ausgeformt ist, in dem ein korrespondierend ausgeformter Falz (21) des Deckelungselements (2) den Lufteinlasskanal (12) und den Auslasskanal (13) abdichtend verschließend aufgenommen ist, wobei der Pulverinhalator (1) ein Anheb-Element aufweist, mittels dessen der Falz (21) aus dem Sperrkanal (18) anhebbar ist.

8. Einzeldosis-Pulverinhalator nach Anspruch 7,
**dadurch gekennzeichnet, dass**
das Anheb-Element
- zumindest ein vorbestimmter und markierter Druck-, Knick- oder Zugpunkt (D,Z) an dem Inhalatorgehäuse ist,
oder
- ein Zugband (5) ist, das sich durch den Lufteinlasskanal (12) und/oder den Auslasskanal (13) und quer durch den Sperrkanal (18) zwischen dem Falz (21) und dem Gehäuseteil (1) erstreckt, und das beidenends einen Griffabschnitt (52) oder an einem Ende einen Griffabschnitt (52) und an dem anderen Ende (50) an einer Ankerstruktur (17') des Inhalatorgehäuses verankert ist,
oder
- durch die Stopfenelemente (4) gebildet wird,
oder
- eine lose in der Medikamentenkammer angeordnete Struktur ist, die bevorzugt der Desagglomerator ist.

9. Einzeldosis-Pulverinhalator nach zumindest einem der Ansprüche 2 bis 8,
**dadurch gekennzeichnet, dass**
der Kunststoff
- biologisch abbaubar ist, und/oder
- einen Marker enthält.

10. Einzeldosis-Pulverinhalator nach zumindest einem der Ansprüche 2 bis 9,
**dadurch gekennzeichnet, dass**
der Kunststoff ein antiseptischer und/oder antimikrobieller Kunststoff ist,
oder dass
zumindest der Lufteinlasskanal (12), die Medikamentenkammer (11) und der Auslasskanal (13) mit einem antiseptischen und/oder antimikrobiellen Beschichtung versehen sind.

11. Einzeldosis-Pulverinhalator (1) nach zumindest einem der Ansprüche 1 bis 10,
**dadurch gekennzeichnet, dass**
die Medikamentenkammer (11) durch zumindest eine Trennwand (11b) in zumindest zwei Teilkammern (11a) unterteilt ist, oder
In dem Gehäuseteil (1) zwei oder mehr Medikamentenkammern (11) ausgeformt sind, die parallel nebeneinander mit jeweils einem Lufteinlasskanal (12) und einem Auslasskanal (13) oder In Serie hintereinander angeordnet sind, wobei der Lufteinlasskanal (12) zu der ersten Medikamentenkammer (11) führt und der Auslasskanal (13) sich von der letzten Medikamentenkammer (11) erstreckt und die Medikamentenkammern (11) über einen weiteren Kanal miteinander verbunden sind.

12. Einzeldosis-Pulverinhalator (1) nach Anspruch 11,
**dadurch gekennzeichnet, dass**
in dem geformten Deckelungselement (2)
- an dem Druckelement (22) jeweils zumindest ein Durchstechelement (23) für jede in dem Gehäuseteil (1) ausgebildete Teilkammer (11a) vorgesehen ist, oder
- für jede in dem Gehäuseteil (1) ausgebildete Medikamentenkammer (11) jeweils ein elastisches Druckelement (22) mit dem zumindest einen Durchstechelement (23) vorgesehen ist.

13. Verfahren zur Herstellung eines Einzeldosis-Pulverinhalators nach zumindest einem der Ansprüche 2 bis 12,
**umfassend die Schritte**
- Herstellen des Gehäuseteils (1) durch Spritzgießen aus Kunststoff oder bevorzugt durch Tiefziehen einer Kunststofffolie,
- Befüllen der Medikamentenkammer (11) mit einer Dosis eines pulverförmigen Medikaments,
- Verschließen der Medikamentenkammer (11) mit einem Folienelement (3), durch zwei Stopfenelemente (4) und/oder durch einen in einem Sperrkanal (18) aufgenommenen Falz (21) des Deckelungselements (2), und
- Ausschneiden des flächigen und im Wesentlichen planaren Deckelungselements (2) oder Herstellen des geformten Deckelelements (2) durch Spritzgießen aus Kunststoff oder bevorzugt durch Tiefziehen einer Kunststofffolie,
- Anfügen des Deckelungselements (2) an dem Gehäuseteil (1), wobei das Verfahren in einer einzigen Vorrichtung durchgeführt wird.

## Claims

1. A single-dose powder inhaler composed of an inhaler housing which has a housing part (1) in which at least one medicament chamber (11) having a dose of a pulverulent medicament is formed, and wherein the inhaler housing has an outlet opening (13') and an outlet duct (13) which extends from the medicament chamber (11) to the outlet opening (13'),
**characterized in that**
the inhaler housing is in two parts and has a housing part (1) and a covering element (2) which, joined together with the housing part (1), forms the inhaler housing, wherein
the outlet duct (13) is formed in the housing part (1),
and **in that**
- an air inlet opening (12') on a side of the medicament chamber (11) remote from the outlet opening (13'), and
- an inlet duct (12) which extends from the air inlet opening (12') to the medicament chamber (11), wherein the outlet duct (13), the medicament chamber (11) and the inlet duct (12) define an axis,
are formed in the housing part (1),
wherein air-guiding, turbulence-inducing and/or deflecting structures (15, 16, 17) are formed in the air inlet duct (12), in the medicament chamber (11) and in the outlet duct (13), and
the inlet duct (12) narrows from the air inlet opening (12') to the medicament chamber (11) in two directions perpendicularly to the axis defined by the outlet duct (13), the medicament chamber (11) and the inlet duct (12), and the outlet duct (13) widens from the medicament chamber (11) in the direction of the outlet opening (13') in two directions perpendicularly to the axis defined by the outlet duct (13), the medicament chamber (11) and the inlet duct (12).

2. The single-dose powder inhaler as claimed in claim 1,
**characterized in that**
the housing part (1) is an injection-molded plastics part or preferably is shaped, particularly preferably thermoformed, from a pharmaceutically compliant, preferably single-type plastic film, wherein the covering element (2)
- is formed in an extensive and substantially planar manner and bounds the inlet duct (12), the medicament chamber (11) and the outlet duct (13),
or
- is formed in a manner corresponding to the housing part (1), at least in the region of the inlet duct (12) and of the outlet duct (13),
and consists of a plastic film, aluminum foil or composite film,
and/or
at least one inhaler housing component, namely the housing part (1) and/or covering element (2), is transparent.

3. The single-dose powder inhaler as claimed in claim 1 or 2,
**characterized in that**
the outlet opening (13') is formed as a mouthpipe or is connected to a nose nozzle (14),
and/or
a cross section of the air inlet duct (12) at the medicament chamber (11) is smaller than a cross section of the outlet duct (13) at the medicament chamber (11).

4. The single-dose powder inhaler as claimed in at least one of claims 1 to 3,
**characterized in that**
at least one loose deagglomerator is arranged in the medicament chamber (11).

5. The single-dose powder inhaler as claimed in at least one of claims 1 to 4,
**characterized in that**
a film element (3) with a closure tab (30) closes the medicament chamber (11).

6. The single-dose powder inhaler as claimed in claim 5,
**characterized in that**
the film element (3) has at least one pull-off tab (32) which, starting from the closure tab (30), extends out of the air inlet opening or outlet opening (12', 13') from one side of the medicament chamber (11) through the opposite air inlet duct or outlet duct (12, 13), or the covering element (2) is formed, on the opposite side from the medicament chamber (11), into a resilient pressure element (22) having at least one piercing element (23) for perforating the closure tab (30) when pressure is exerted on the pressure element (22).

7. The single-dose powder inhaler as claimed in at least one of claims 1 to 4,
**characterized in that**
- the medicament chamber (11) is closed by two plug elements (4), wherein each plug element (4) has a plug portion (40), which is arranged in a portion (120, 130), adjacent to the medicament chamber (11), of the air inlet duct (12) and of the outlet duct (13), and a pull-off tab (42), which extends out of the air inlet opening (12') and the outlet opening (13'), respectively,
or
- a blocking duct (18) is formed in the housing part (1) on both sides of the medicament chamber (11) at right angles to the air inlet duct (12) and the outlet duct (13), a correspondingly formed fold (21) of the covering element (2) being received in said blocking duct (18) in a manner closing the air inlet duct (12) and the outlet duct (13) in a sealed manner, wherein the powder inhaler (1) has a lifting element by means of which the fold (21) is able to be lifted out of the blocking duct (18).

8. The single-dose powder inhaler as claimed in claim 7,
**characterized in that**
the lifting element
- is at least one predetermined and marked pressure, bending or pulling point (D, Z) on the inhaler housing,
or
- is a pull tape (5) which extends through the air inlet duct (12) and/or the outlet duct (13) and transversely through the blocking duct (18) between the fold (21) and the housing part (1), and which a grip portion (52) at both ends or a grip portion (52) at one end and is anchored to an anchoring structure (17') of the inhaler housing at the other end (50),
or
- is formed by the plug elements (4),
or
- is a structure arranged loosely in the medicament chamber, said structure preferably being the deagglomerator.

9. The single-dose powder inhaler as claimed in at least one of claims 2 to 8,
**characterized in that**
the plastic
- is biodegradable, and/or
- contains a marker.

10. The single-dose powder inhaler as claimed in at least one of claims 2 to 9,
**characterized in that**
the plastic is an antiseptic and/or antimicrobial plastic,
or **in that**
at least the air inlet duct (12), the medicament duct (11) and the outlet duct (13) are provided with an antiseptic and/or antimicrobial coating.

11. The single-dose powder inhaler (1) as claimed in at least one of claims 1 to 10,
**characterized in that**
the medicament chamber (11) is subdivided into at least two subchambers (11a) by at least one partition wall (11b), or
two or more medicament chambers (11) are formed in the housing part (1), which are arranged in parallel alongside one another in each case with an air inlet duct (12) and an outlet duct (13) or are arranged in series with one another, wherein the air inlet duct (12) leads to the first medicament chamber (11) and the outlet duct (13) extends from the last medicament chamber (11) and the medicament chambers (11) are connected together via a further duct.

12. The single-dose powder inhaler (1) as claimed in claim 11,
**characterized in that**,
in the shaped covering element (2),
- at least one piercing element (23) for each subchamber (11a) formed in the housing part (1) is provided in each case on the pressure element (22), or
- a resilient pressure element (22) having the at least one piercing element (23) is provided in each case for each medicament chamber (11) formed in the housing part (1).

13. A method for producing a single-dose powder inhaler as claimed in at least one of claims 2 to 12,
**comprising the steps of**
- producing the housing part (1) from plastic by injection-molding or preferably by thermoforming a plastic film,
- filling the medicament chamber (11) with a dose of a pulverulent medicament,
- closing the medicament chamber (11) with a film element (3), by two plug elements (4) and/or by a fold (21), received in a blocking duct (18), of the covering element (2), and
- cutting out the extensive and substantially planar covering element (2) or producing the shaped cover element (2) from plastic by injection-molding or preferably by thermoforming a plastic film,
- attaching the covering element (2) to the housing part (1),
wherein the method is carried out in a single device.

## Revendications

1. Inhalateur de poudre à dose unitaire comprenant un corps d'inhalateur, qui présente une partie de boîtier (1), dans laquelle est aménagée au moins une chambre à médicament (11) avec une dose d'un médicament sous forme pulvérulente, le corps d'inhalateur possédant une ouverture de sortie (13') et un canal de sortie (13), qui s'étend de la chambre à médicament (11) à l'ouverture de sortie (13'),
**caractérisé en ce que**
le corps d'inhalateur est composé de deux parties et présente une partie de boîtier (1) et un élément de recouvrement (2) qui, assemblé avec la partie de boîtier (1), forme le corps d'inhalateur, dans lequel
le canal de sortie (13) est aménagé dans la partie de boîtier (1),
et que
sont formés dans la partie de boîtier (1)
- une ouverture d'entrée d'air (12') sur un côté de la chambre à médicament (11) à l'opposé de l'ouverture de sortie (13') et
- un canal d'entrée (12) qui s'étend de l'ouverture d'entrée d'air (12') vers la chambre à médicament (11), le canal de sortie (13), la chambre à médicament (11) et le canal d'entrée (12) définissant un axe,
l'ensemble étant conçu de sorte que des structures de guidage de l'air, de tourbillonnement de l'air et/ou de déviation de l'air (15, 16, 17) sont formées dans le canal d'entrée d'air (12), dans la chambre à médicament (11) et dans le canal de sortie (13), et
que le canal d'entrée (12) se rétrécit dans deux directions de l'ouverture d'entrée d'air (12') à la chambre à médicament (11) verticalement par rapport à l'axe défini par le canal de sortie (13), la chambre à médicament (11) et le canal d'entrée (12) et que le canal de sortie (13) s'élargit dans deux directions de la chambre à médicament (11) vers l'ouverture de sortie (13') verticalement par rapport à l'axe défini par le canal de sortie (13), la chambre à médicament (11) et le canal d'entrée (12).

2. Inhalateur de poudre à dose unitaire selon la revendication 1,
**caractérisé en ce que**
la partie de boîtier (1) est une pièce formée par injection de matière plastique ou, de préférence, qu'elle est formée à partir d'une feuille de matière plastique de même nature et conforme aux exigences du secteur pharmaceutique, et de manière particulièrement préférée, qu'elle est thermoformée, l'élément de recouvrement (2) étant
- formé par une surface essentiellement plane, de sorte qu'il limite le canal d'entrée (12), la chambre à médicament (11) et le canal de sortie (13), ou
- qu'au moins dans la zone du canal d'entrée (12) et du canal de sortie (13), il est formé en correspondance avec la partie de boîtier (1),
et se compose d'une feuille de matière plastique, d'aluminium ou composite,
et/ou
qu'au moins un composant du corps d'inhalateur, la partie de boîtier (1) et/ou l'élément de recouvrement (2), est transparent.

3. Inhalateur de poudre à dose unitaire selon la revendication 1 ou 2
**caractérisé en ce que**
l'ouverture de sortie (13') est formée en tant qu'embout buccal ou qu'elle est reliée à une trompe nasale (14),
et/ou
qu'une section du canal d'entrée d'air (12) à la chambre à médicament (11) est plus petite qu'une section du canal de sortie (13) à la chambre à médicament (11).

4. Inhalateur de poudre à dose unitaire selon au moins une des revendications 1 à 3, **caractérisé en ce que**
au moins un désagglomérateur libre est agencé dans la chambre à médicament (11).

5. Inhalateur de poudre à dose unitaire selon au moins une des revendications 1 à 4, **caractérisé en ce que**
un élément en feuille (3) avec une languette de fermeture (30) obture la chambre à médicament (11).

6. Inhalateur de poudre à dose unitaire selon la revendication 5,
**caractérisé en ce que**
l'élément en feuille (3) présente au moins une languette à tirer (32) qui, en partant de la languette de fermeture (30), s'étend d'un côté de la chambre à médicament (11) à travers le canal d'entrée d'air d'en face ou le canal de sortie (12, 13) d'en face à partir de l'ouverture d'entrée d'air ou de l'ouverture de sortie (12', 13'), ou que l'élément de recouvrement (2) opposé à la chambre à médicament (11) est moulé sous forme d'un élément de pression (22) avec au moins un élément de perforation (23) pour la perforation de la languette de fermeture (30) sous l'action d'une pression sur l'élément de pression (22).

7. Inhalateur de poudre à dose unitaire selon au moins une des revendications 1 à 4,
**caractérisé en ce que**
- la chambre à médicament (11) est fermée par deux éléments de bouchage (4), chaque élément de bouchage (4) comportant une section de bouchage (40), agencée dans une section (120, 130) voisine de la chambre à médicament (11) du canal d'entrée d'air (12) et du canal de sortie (13), et une languette à tirer (42), qui s'étend respectivement hors de l'ouverture d'entrée d'air (12') et de l'ouverture de sortie (13'),
ou
- qu'est formé dans la partie de boîtier (1) des deux côtés de la chambre à médicament (11), perpendiculairement au canal d'entrée d'air (12) et au canal de sortie (13), un canal de blocage (18), dans lequel est logé un pli (21) formé en correspondance de l'élément de recouvrement (2) de sorte que ledit pli ferme de manière étanche le canal d'entrée d'air (12) et le canal de sortie (13), l'inhalateur de poudre (1) présentant un élément de levage, qui permet de soulever le pli (21) hors du canal de blocage (18).

8. Inhalateur de poudre à dose unitaire selon la revendication 7,
**caractérisé en ce que**
l'élément de levage
- est au moins un point prédéfini et marqué de pression, de pliage ou de traction (D, Z) au corps de l'inhalateur,
ou
- est une bande de traction (5), qui s'étend à travers du canal d'entrée d'air (12) et/ou du canal de sortie (13) et transversalement à travers le canal de blocage (18) entre le pli (21) et la partie de boîtier (1), et qui possède aux deux extrémités une partie de préhension (52) ou qui possède une partie de préhension (52) à une extrémité et est ancrée à l'autre extrémité (50) à une structure d'ancrage (17') du corps d'inhalateur,
ou
- est formé par les éléments de bouchage (4),
ou
- est une structure libre logée dans la chambre à médicament, ladite structure libre étant de préférence le désagglomérateur.

9. Inhalateur de poudre à dose unitaire selon au moins une des revendications 2 à 8,
**caractérisé en ce que**
la matière plastique
- est biodégradable, et/ou
- comporte un marqueur.

10. Inhalateur de poudre à dose unitaire selon au moins une des revendications 2 à 9, **caractérisé en ce que**
la matière plastique est une matière plastique antiseptique et/ou antimicrobienne, ou que
au moins le canal d'entrée d'air (12), la chambre à médicament (11) et le canal de sortie (13) comportent un revêtement antiseptique et/ou antimicrobien.

11. Inhalateur de poudre à dose unitaire (1) selon au moins une des revendications 1 à 10,
**caractérisé en ce que**
la chambre à médicament (11) est subdivisée en au moins deux chambres partielles (11a) par une cloison de séparation (11b), ou
que sont formées dans la partie de boîtier (1) deux ou plusieurs chambres à médicament (11), qui sont agencées parallèlement côte-à-côte avec respectivement un canal d'entrée d'air (12) et un canal de sortie (13) ou en série d'affilée, le canal d'entrée d'air (12) conduisant à la première chambre à médicament (11) et le canal de sortie (13) s'étendant à partir de la dernière chambre à médicament (11) et les chambres à médicament (11) étant reliées entre elles par un canal supplémentaire.

12. Inhalateur de poudre à dose unitaire (1) selon la revendication 11,
**caractérisé en ce que**
dans l'élément de recouvrement (2) formé
- est prévu à l'élément de pression (22) respectivement au moins un élément de perforation (23) pour chaque chambre partielle (11a) formée dans la partie de boîtier (1), ou
- que pour chaque chambre à médicament (11) formée dans la partie de boîtier (1) est prévu respectivement un élément de pression (22) élastique avec au moins un élément de perforation (23).

13. Procédé de fabrication d'un inhalateur de poudre à dose unitaire selon au moins une des revendications 2 à 12,
**comprenant les étapes**
- fabrication de la partie de boîtier (1) par moulage par injection de matière plastique ou de préférence par thermoformage d'une feuille en matière plastique,
- remplissage de la chambre à médicament (11) avec une dose d'un médicament pulvérulent,
- obturation de la chambre à médicament (11) avec un élément en feuille (3), par deux éléments de bouchage (4) et/ou par un pli (21) de l'élément de recouvrement (2) logé dans un canal de blocage (18), et
- découpage de l'élément de recouvrement (2) formé par une surface essentiellement plane ou fabrication de l'élément de recouvrement (2) formé par moulage par injection de matière plastique ou de préférence par thermoformage d'une feuille en matière plastique,
- rapporter l'élément de recouvrement (2) à la partie de boîtier (1),
le procédé étant exécuté dans un seul dispositif.
